# EUROPEAN PATENT APPLICATION

(11) **EP 4 645 334 A1**
(43) Date of publication of application: **05.11.2025**
(21) Application number: 24174174.3
(22) Date of filing: 03.05.2024
(51) Int. Cl.: G16H 40/63, G16H 10/60, G16H 15/00, G16H 50/20, G16H 50/30, G16H 50/70, G06F 3/01

(54) **DISPLAYING MEDICAL DATA**

(71) Applicant: Koninklijke Philips N.V., 5656 AG Eindhoven (NL)
(72) Inventor: KUHLMANN, Daan, Eindhoven (NL); VAN LIESHOUT, Ron Martinus Laurentius, Eindhoven (NL); BINGLEY, Peter, 5656AG Eindhoven (NL); BULUT, Murtaza, Eindhoven (NL); HUIJBREGTS, Laurentia Johanna, Eindhoven (NL); BRANS, Harold Johannes Antonius, Eindhoven (NL); BUIL, Vincentius Paulus, Eindhoven (NL)
(74) Representative: Philips Intellectual Property & Standards

(57) **Abstract**

Disclosed concepts aim to provide schemes, solutions, concepts, designs, methods, and systems pertaining to displaying medical data, for example on a patient monitor. The graphical representation of medical data of a subject is modified based on whether a gazing pattern of the user differs from an expected gazing pattern. If the pattern of their observation (e.g., the order that the user observes parts of the medical data, the amount of time dwelling on certain parts of the medical data, etc.) differs significantly from that of an expected pattern of observation, then the graphical representation may be modified. The modification may draw attention of the user to overlooked or underappreciated parts of the medical data, thus improving an interaction of the user with the display.

## Description

### FIELD OF THE INVENTION

The present invention relates to the field of subject management, and more particularly to displaying medical data of one or more subjects.

### BACKGROUND OF THE INVENTION

Healthcare professionals often assess the condition of one or more subjects using patient monitors, which present medical data of one or more subjects. Patient monitors may represent the medical data graphically, in that the medical data may be presented in the form of graphs, charts, trend indicators, numbers, text, images, and video. The presented data may include vital signs (e.g., heart rate, respiration rate, and blood pressure), advanced hemodynamic parameters (e.g., cardiac output, stroke volume, and systemic vascular resistance), haematological parameters (e.g., red and white blood cell counts and haemoglobin concentration), and analyte concentrations (e.g., glucose or lactate concentrations in whole blood, plasma, or serum). Similarly, healthcare professionals interact with electronic medical records (EMR) of subjects through EMR system interfaces, which also represent the medical data graphically.

Different users may observe presented medical data in different ways. For example, there is an inherent difference between how a novice healthcare professional, and an expert healthcare professional examine the same medical data. As a result, different users may draw different conclusions due to observing parts of the medical data for differing amounts of time. That is, some users may over-fixate on some parts of the medical data, whilst missing other parts of the medical data, thus potentially resulting in different conclusions being drawn.

More particularly, cognitive bias, such as tunnel vision, is a problem that novice and expert users may both experience. Novice users may be very focused on what they are familiar with, and might not be aware of certain events that can contribute to a certain problem. Expert users may have seen similar medical data many times previously, which may lead them to complacency and thus missing certain values. Ultimately, this may lead to a difference in subject management depending on the observing user. For example, different users may perform measurements, provide diagnoses, initiate communication with clinicians, update subject medical records, and/or update device settings, differently responsive to observing the same medical data. However, it is in the subject's interest that subject management is consistent, and that errors are minimized.

Therefore, there exists a need for means of presenting medical data in such a way that different users may gather similar information and therefore are more likely to reach a similar conclusion.

### SUMMARY OF THE INVENTION

The invention is defined by the claims.

According to examples in accordance with the invention, there is provided a method for displaying medical data. The method comprises:
detecting a gazing pattern describing a time-varying eye gaze direction of a user observing a graphical representation of the medical data;
determining a deviation of the detected gazing pattern from an expected gazing pattern describing an expected time-varying eye gaze direction of the user observing the graphical representation; and
modifying, based on the deviation of the detected gazing pattern, the graphical representation of the medical data.

There are proposed concepts for modifying the presentation of medical data on, for example, a patient monitor. Specifically, the graphical representation of medical data of a subject is modified based on whether a gazing pattern of the user differs from an expected gazing pattern. That is, a user observes the displayed medical data. If the pattern of their observation (e.g., the order that the user observes parts of the medical data, the amount of time dwelling on certain parts of the medical data) differs significantly from that of an expected pattern of observation, then the graphical representation may be modified. The modification may draw attention of the user to overlooked or underappreciated parts of the medical data, thus improving an interaction of the user with the display.

By way of example, if the user observes the medical data but fails to spend adequate time observing a particular part of the medical data that may be of relevance, such as an elevated urine output value, then the graphical representation may be modified to draw attention of the user to that elevated urine output value. Equally, if the user is expected to be performing a certain clinical workflow, but the detected gaze direction indicates that they have missed a step (e.g., forget to check airways of the subject before checking breathing of the subject), then the graphical representation of the medical data presented to the user may be modified to prompt performance of the missed step.

Indeed, when any particular user observes a graphical representation of medical data (e.g., on a patient monitor), they will focus on the parts of the medical data that they deem to be more relevant for a longer amount of time, and may only glance or entirely disregard other parts of the medical data. Other users may focus on, or disregard, different parts of the medical data. Nevertheless, there may be certain parts of the medical data that need to be acknowledged (e.g., due to their high clinical relevance, or because checking them forms part of a prescribed workflow of the user). Thus, if it is detected that the detected gazing pattern of the user differs from an expected gazing pattern, then this may form the reasoning behind modification of the representation of the medical data to attract the attention of the user. Accordingly, it may be ensured that every user conforms with an expected gazing pattern - potentially leading to improved patient outcomes.

To be clear, the medical data may include medical data of a single subject, such as on a personal patient monitor. Alternatively, the medical data may include medical data of a plurality of subjects, such as on a central patient monitor hub in a particular department. The gazing pattern describes how the user looks at the graphical representation of the medical data, and therefore may indicate the time spent looking at particular parts of the medical data, and/or an order that the user looked at parts of the medical data. The graphical representation includes any visual depiction of the medical data, including raw text, images, video, graphs, charts, trend indicators, magnitude indicators, raw numbers, colors, motion, position, size, font etc.

Modifying the graphical representation may encompass emphasizing or lessening, reformatting, or reordering parts of the medical data. Modifying the graphical representation includes any way of visually altering the medical data so that the gaze of the user may be redirected to or from parts of the medical data, to change an order in which the user observes the medical data, and/or a time taken by the user to observe particular parts of the medical data. Not only may this save time of the user as they may intuitively understand which parts of the medical data to focus upon, this may result in improved situational awareness, and in turn improved conclusions and user-independent subject management actions . Specifically, it may overcome tunnel-vision related issues that are learned or habitually formed (i.e., it may avoid cognitive bias).

In some embodiments, the method may further comprise obtaining a plurality of template gazing patterns, each template gazing pattern describing an expected time-varying eye gaze direction of a user observing medical data; and determining the expected gazing pattern based on the plurality of template gazing patterns.

In other words, the method may comprise retrieving a plurality of template gazing patterns from a repository of pre-generated template gazing patterns. Each of the template gazing patterns may describe a different way in which the user may observe medical data. For example, some of the template gazing patterns may reflect the expected gaze of the user as they observe medical data according to different workflows. Other template gazing patterns may reflect the expected gaze of the user as they observe medical data of a subject with a certain condition. The template gazing patterns may further be associated with users of different experience levels and/or roles, or may reflect users in different contexts (i.e., surrounding environments and circumstances).

Each of the plurality of template gazing patterns may be pre-programmed/pre-generated, and each may reflect an expected gazing pattern of a user when observing certain sets of medical data (e.g., medical data associated with a certain subject condition, or medical data associated with an alarm condition). In some cases, each template gazing pattern may also be derived, for example, using a machine learning algorithm adapted to generate template gazing patterns based on sets of medical data.

The expected gazing pattern is based on one or more of the template gazing patterns. In a simple example, the expected gazing pattern may be selected from one of the plurality of gazing patterns. In other embodiments, one or more of the template gazing patterns may be edited to determine the expected gazing patterns. This provides a simple manner of providing an expected gazing pattern of the user.

More specifically, the method may further comprise determining the expected gazing pattern based on a status and/or condition of a subject associated with the medical data.

The target way that the user observes the medical data may be heavily dependent upon the status or condition of the subject of the medical data. For example, heart-related medical data (e.g., heart rate, heart rate variability, etc) may be expected to be viewed for longer periods of time if the subject of the medical data had recently experienced cardiac arrest. Further, if a subject is in a deteriorating condition, then more time may be expected to be spent observing trends of physiological parameters than the present values of physiological parameters.

Accordingly, by determining the expected gazing pattern based on the present status/condition of the subject, the user may be prompted to observe more relevant medical data by the modification of the graphical representation.

According to one method, determining the expected gazing pattern may comprise:
obtaining a plurality of template gazing patterns, each template gazing pattern describing an expected time-varying eye gaze direction of a user observing medical data of a subject with a respective status and/or condition;
for each of the plurality of template gazing patterns, comparing the respective status and/or condition associated with the template gazing pattern with the status and/or condition of the subject associated with the medical data to determine a similarity value; and
selecting at least one of the plurality of template gazing patterns based on similarity values of each of the template gazing patterns; and
providing the expected gazing pattern based on the selected template gazing patterns.

As a result, a template gazing pattern closely corresponding to an expected gazing pattern of the user given a subject condition/status may be determined. An appropriate assessment of the detected gazing pattern may thus be performed.

Alternatively, determining the expected gazing pattern may comprise generating, with a gazing pattern prediction model, the expected gazing pattern based on the status and/or condition of the subject associated with the medical data.

In other words, another way in which the expected gazing pattern may be obtained is to generate the gazing pattern. This may comprise determine an appropriate length of time that a user should observe certain parts of the medical data, or an order by which parts of the medical data should be observed given the subject condition/status. This may be more computationally complex than retrieving and optionally editing a template gazing pattern, but may be more appropriate for the given medical data.

The method may further comprise analyzing the medical data to determine the status and/or condition of a subject associated with the medical data.

It has also been realized that the status and condition of the subject may be determined from the medical data. Accordingly, a self-contained system that determines the likely condition and/or status of the subject, and determines an expected gazing pattern based on the condition of the status of the subject may be provided. That is, this means that external input is not required to determine an expected gazing pattern appropriate for the status and/or condition of the subject.

In some embodiments, the method may further comprise determining the expected gazing pattern based on one or more characteristics of the user. In particular, the one or more characteristics may comprise a role, an experience level, a user identifier, and a user-subject familiarity.

It has been realized that certain characteristics of the user are another factor that impacts the expected gazing pattern of the user. For example, a user that has the role of a nurse may be expected to observe the medical data in such a way so as to check whether there is a change in a status of the subject, whereas a user that has a role as a doctor may be expected to observe the medical data in such a way so as to determine a diagnosis. As a further example, a certain user may have observed the medical data recently so that the user may focus on parts of the medical data that have changed in the interim, whereas another user observing the medical data for the first time may be expected to spend time looking at all parts of the medical data.

Thus, by accounting for characteristics of the user, a more appropriate expected gazing pattern may be determined. In turn, this may lead to modification of the graphical representation that is more beneficial to the given user.

In this case, determining the expected gazing pattern may comprise:
obtaining a plurality of template gazing patterns, each template gazing pattern describing an expected time-varying eye gaze direction of a different user observing medical data, each different user having one or more characteristics;
for each of the plurality of template gazing patterns, comparing the respective characteristics of the user associated with the template gazing pattern with the characteristic of the user to determine a similarity value; and
selecting at least one of the plurality of template gazing patterns based on similarity values of each of the template gazing patterns; and
providing the expected gazing pattern based on the selected template gazing patterns.

As a result, a template gazing pattern closely corresponding to an expected gazing pattern of the user given characteristics of the user may be determined. An appropriate assessment of the detected gazing pattern may thus be performed.

The method may further comprise analyzing the detected gazing pattern to determine the one or more characteristics of the user.

It has also been realized that certain characteristics of the user may be determined from the way in which the user observes the data. For example, a user who quickly scans the medical data without hesitation may be assessed to be an experienced user, whilst a user that hesitates and continually re-observes part of the data may be a novice user. This may provide a means for determine an appropriate expected gaze pattern without requiring user input or input from other sources.

In some embodiments, the method may further comprise determining the expected gazing pattern based on a contextual information associated with the user. In particular, wherein the contextual information comprises environmental information describing a surrounding environment of the user, or time-related information describing time-based parameters associated with the user.

Yet another factor that may impact the expected gaze pattern will be the context in which the user observes the data. It may be the case that the user is in a rush and therefore a thorough assessment of the medial data by observing all parts of the medical data may not be possible. In this case, the expected gazing pattern may be adjusted to reflect this - perhaps ensuring that the user observes only the most important parts of the medical data. Furthermore, if the user is in a busy or distracting environment, they expected gazing pattern may be adjusted accordingly. As a result, a realistic expected gazing pattern may be determined, in turn leading to an appropriate modification of the graphical representation of medical data.

Further embodiments of the invention may comprise a step of analyzing the deviation to determine a clinical relevance of the deviation, and wherein the graphical representation of the medical data is modified responsive to the clinical relevance satisfying a predetermined condition.

As yet a further condition, the graphical representation of the medical data may only be modified if the deviation of the gaze pattern of the user is deemed to be clinically relevant. That is, if the user deviates from the expected gaze pattern, but this deviation has no clinical relevance, then the graphical representation may not be modified. This prevents unnecessary modification when the user has deviated in only immaterial ways.

For example, if the user was expected to observe part of the medical data for a certain period of time, but the user exceeds this period of time, then no modification may be necessary. Similarly, if the user was expected to observe parts of the medical data in a certain sequence, but has instead slightly altered the sequence, there may be no need for modification. In contrast, if the user fails to observe a key part of the medical data, or observed parts of the medical data in such an order so they must not have adequately performed a certain workflow, then modification of the graphical representation may be necessary.

Accordingly, this ensures that the modification only occurs when it is clinically relevant to do so, and therefore the impact of the modification on the behavior of the user may be improved. Indeed, if the modification is often superfluous, then the user may learn to simply ignore the modification. This embodiment may help to mitigate this issue.

In addition, the method may comprise:
detecting, responsive to modifying the graphical representation, a prompted gazing pattern describing an eye gaze direction as a function of time of the user observing the modified graphical representation; and
determining a deviation of the prompted gazing pattern from a target gazing pattern describing a target eye gaze direction of the user responsive to the modification; and
modifying, based on the deviation of the prompted gazing pattern, the graphical representation of the medical data.

To paraphrase, the graphical representation of the medical data may be modified again if the resultant gazing pattern of the user (i.e., prompted gazing pattern) deviates from a target gazing pattern due to the previous modification. For example, if the user still does not observe part of the medical data, or continues to observe the medical data in a wrong order, then the graphical representation may be modified in a different way, or the modification may be more greatly emphasized in an attempt to prompt appropriate user behavior.

Thus, there is provided a feedback loop to ensure that the user observes the medical data in a desired manner. This may result in improved user observation of the medical data.

According to some embodiments of the invention, the detected gazing pattern and the expected gazing pattern may each be represented as heat maps, wherein each region of the heatmap identifies a different region of the graphical representation, and wherein a value associated with each region describes a length of time that the user has observed the respective region of the graphical representation.

Heatmaps present one straightforward way for providing the gazing patterns. Nevertheless, the gazing patterns may simply be provided in the form of values, such as a list of parts of the medical data and corresponding observation times, or a list of parts of the medical data in order of sequence of observation.

According to examples in accordance with another aspect of the invention, there is provided a computer program comprising computer program code means adapted, when said computer program is run on a computer, to implement a disclosed method for displaying medical data according to any disclosed embodiment.

According to further examples in accordance with an aspect of the invention, there is provided a patient monitor, comprising:
a user interface configured to display a graphical representation of medical data;
a camera system configured to detect a gazing pattern describing an eye gaze direction as a function of time of a user observing a graphical representation of the medical data; and
a processor configured to determine a deviation of the detected gazing pattern from an expected gazing pattern, and modify, based on the deviation of the detected gazing pattern, the graphical representation of the medical data.

These and other aspects of the invention will be apparent from and elucidated with reference to the embodiments described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a flow diagram of a method for displaying medical data according to an embodiment of the invention;
Fig. 2 is a flow diagram of a method for determining an expected gazing pattern according to an aspect of the invention;
Fig. 3 is a simplified block diagram of a patient monitor according to another embodiment of the invention; and
Fig. 4 is a simplified block diagram of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. The mere fact that certain measures are recited in mutually different dependent claims does not indicate that a combination of these measures cannot be used to advantage.

It should be understood that the Figures are merely schematic and are not drawn to scale. It should also be understood that the same reference numerals are used throughout the Figures to indicate the same or similar parts.

Disclosed concepts aim to provide schemes, solutions, concepts, designs, methods, and systems pertaining to displaying medical data, for example on a patient monitor. The graphical representation of medical data of a subject is modified based on whether a gazing pattern of the user differs from an expected gazing pattern. If the pattern of their observation (e.g., the order that the user observes parts of the medical data, the amount of time dwelling on certain parts of the medical data, etc.) differs significantly from that of an expected pattern of observation, then the graphical representation may be modified. The modification may draw attention of the user to overlooked or underappreciated parts of the medical data, thus improving an interaction of the user with the display.

By way of explanation, typically there is a difference between how different users go through, or observe, medical data on a monitor. Some users may experience tunnel vision, in which they only focus on parts of the medical data that they expect to be of relevance, or to which they are familiar. As a result, there may be a difference in conclusions drawn by different users from the same medical data. Ultimately, this could lead a difference between diagnosis and/or treatment of subjects. It is thus in the interest of the subject(s) that the conclusions are consistent, and that potential errors are minimized.

The expected gazing pattern of the user observing the medical data may relate to the clinical situation (i.e. situation of the patient(s) described by the medical data), the situation/context of the user, as well as the user themselves (e.g., their role, expertise, experience, etc.). Ultimately, the expected gazing pattern should be such that the user performing the expected gazing pattern should obtain all medical information required for a fully informed conclusion. If the actual gazing pattern of the user deviates from the expected gazing pattern, then this may mean that the user has missed parts of the medical data that are of relevance. This deviation may therefore determine whether action needs to be taken.

The present invention may be suited for displaying medical information on a patient monitor that presents real-time medical data of a subject. In addition, the invention may be particularly suitable for adapting the display of electronic medical records (EMR) by an EMR interface system that presents medical data of the subject. The invention ensures that, in either case, the presented medical data is observed by the user in an expected manner. Should the user observe the medical data in a manner deviating from the expected manner, then the graphical representation of the medical data (e.g., on the patient monitor, on the EMR, or on a mobile device of the user) may be adapted to prompt the user to observe the medical data in a different way. Accordingly, insights drawn from the medical data may be normalized between different users. In turn, this may lead to improved consistency of patient management between different users (whom may be inherently inclined to observe the medical data in differing ways).

Eye gaze tracking is widely used for research purposes. Typically, an eye tracking system comprises one or more cameras, some light sources, and computing capabilities. Algorithms translate the camera feed into data points, potentially with the help of machine learning and advanced image processing. At present, this technology has been used to understand the interaction between users and patient monitor systems. However, it has not been used in a way that incorporates clinical elements, to provide a measurement of desired interaction between the user and the patient monitor system, and/or to optimize the patient monitor system.

Proposed concepts thus combine information regarding the actual/detected gaze pattern of a user viewing a graphical representation of medical data, for example using a patient monitor, and an expected gaze pattern of the user. From the difference, the graphical representation of the medical data can be adapted in a meaningful way. Accordingly, there may be provided 3 key elements:
(i) Detection of the gazing pattern of the user, which describes a time-varying eye gaze direction of a user observing the graphical representation of the medical data. For example, fixation patterns (i.e. milliseconds spent observing a particular part of the medical datala certain part of the patient monitor) of the user may be measured, and the experience of the user with the system can be derived (i.e. fixed vs dwelling and fixation on parts of the medical data).
(ii) Determining/identifying a deviation of the detected gazing pattern from an expected gazing pattern. The expected gazing pattern describes an expected/target time-varying eye gaze direction of the user observing the graphical representation.
   The expected gazing pattern may be based on the values of the medical data, as well as any prior usage of the medical data. In some cases, the expected gazing pattern may be based on a cohort of template gazing patterns from various healthcare providers. For example, if template gazing patterns associated with experienced healthcare providers have a fixation pattern on the urine output value, but the current user has overlooked this then this deviation may be significant.
   In additional and/or alternative cases, the expected gazing pattern may be derived from an expected clinical workflow. In an example use case, the user needs to do an ABCD check on a subject within an ICU in an acute setting. An ABCD approach has a pattern that follows the same steps whenever a subject is critically deteriorating. First the airways (A) of the subject are checked, then the breathing (B), then circulation (C), then disability (D). Accordingly, if the detected gazing pattern of the user does not correspond to an expected gazing pattern corresponding to the ABCD check then this may indicate that key pieces of the medical data have been missed.
(iii) Modifying the graphical representation of the medical data. This is based on a deviation of the detected gazing pattern from the expected gazing pattern. In other words, the detected and expected gazing patterns are compared, and any differences may be used as the basis for modification of the graphical representation of the medical data. For example, if the deviation indicates that the user may have missed a vital value included in the medical data currently displayed, it may alter the graphical representation so as to nudge/prompt the user to look at this specific value.

The graphical representation of the medical data could be modified so that the outcome of the interaction (i.e.., the user observing the medical data) is matched with the most optimal interaction profile. This may aid in maximizing standardization of subject management, and thus reduce errors in medical actions taken in relation to the subject, or even diagnosis of the subject. By attempting to prompt the user to observe the medical data in a particular way, the conclusions drawn by the user may be biased toward the best result, minimizing errors made by the user, and thus helping the subject. The incorporation of said optimization can be done by directly guiding the user by the graphical representation (e.g., the screen flashes at a part of a screen corresponding to the part of the medical data that might have been overseen by the user, the part of the medical data being required for a complete problem assessment).

In some embodiments, whenever the graphical representation is modified, it may be checked if the gazing pattern of the user changes in an expected way. That is, the prompted gazing pattern of the user (i.e., prompted by the graphical representation modification) is compared to an expected gazing pattern induced by the modification. When it is determined that the user's gazing direction has not been altered in the expected manner, other methods of altering the graphical representation may be attempted to prompt the expected change in gazing pattern.

The proposed concepts may further be adapted to specific user characteristics. That is, un a case where the experience level of the user is known, then the expected gazing pattern may reflect this (e.g., the expected gazing pattern reflects a pattern of observation suitable for gathering any information helpful for a novice user to understand the situation, whereas the expected gazing pattern for an expert user may only prescribe that the user observes only essential information). Indeed, a novice user might need more information from the medical data than an expert user to reach a sensible conclusion. Accordingly, the modified graphical representation may prompt a novice user to look at different elements of the medical data than an expert user, thus helping them get a better understanding of the current situation. This can be especially useful in an ICU context where sometimes quick decisions must be made.

To this end, in some embodiments of the invention, the detected gazing pattern may be used to determine characteristics of the user, or even identify the user observing the medical data. The determination may be achieved by using prior gaze pattern recognition (optical identifiers that are derived from the gaze tracking itself), or a camera or RFID tag could be used to determine which user is nearby a screen displaying the medical data, or observing the medical data. In other embodiments, the medical data may be displayed on a virtual augmented, or mixed reality device (such as a VR/AR headset) that is associated with a given user. In this case, the user will be known by association with the device. The user identifier may be used to determine an experience level and/or role the current user has, as each role and experiences have different medical data that they are expected to observe (e.g., difference between nurse and intensivist). The users may also be put in specific buckets 1) novice in role A, 2) expert in role A, 3) novice in role B, 4) expert in role B, 5) novice in role C, etc.

In an extra embodiment, the detected gaze pattern may be used to determine the user alertness, or from extra measurements taken from a same eye-tracking system (e.g., alertness could be determined during the first moments a user is observing the medical data). If a user is alert, they will likely need less time and go directly to the area of the medical data that is expected. If a user is distracted (perhaps due to the activities that they had been doing earlier), they may take more time and they may search through large parts of the medical data to find the right next step. Alertness could also be determined by tracking blinking or eye gaze patterns. If it is noted via the feedback loop that a user is not optimally alert and misses important parts of the medical, this may be used to determine the modification of the graphical representation, or even to alert the user and other users as to their alertness level.

In yet further embodiments, the detected gazing pattern could be extended to cover other devices in the room, such as infusion pumps or a ventilator, to make sure these also get sufficient visual attention from the user. That is, not only medical data may be being observed by the user, but also devices and the subject themselves.

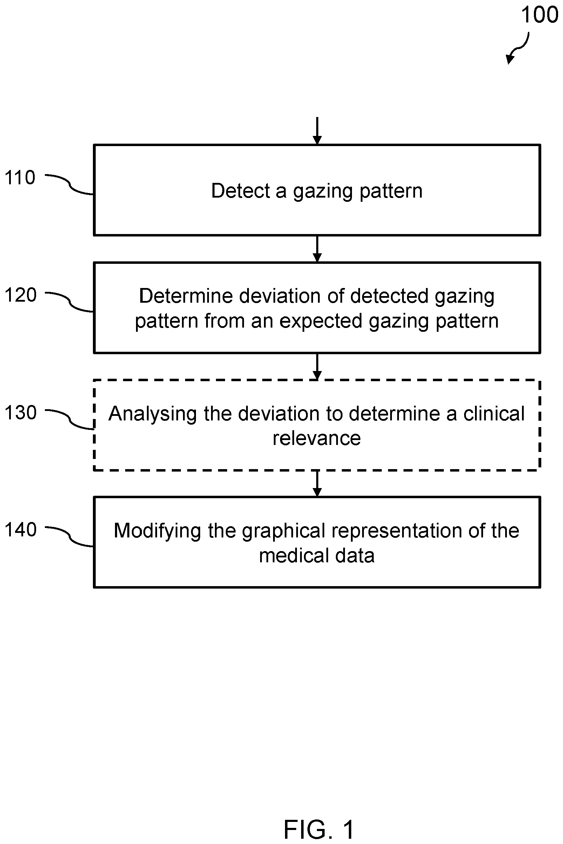

Turning to Fig. 1, there is presented a flow diagram of a method for displaying medical data according to an embodiment of the invention. That is, there is provided a method for presenting medical data, such as medical data of a subject, or medical data relating to a plurality of subjects. The medical data may be displayed on a screen, such as a patient monitor.

In step 110, a gazing pattern of a user is detected. The gazing pattern describes a time-varying eye gaze direction of the user observing a graphical representation of the medical data. The detected gazing pattern may thus describe time spent by the user observing different parts of the medical data, and/or an order according to which the user observed different parts of the medical data. Accordingly, the detected gazing pattern may indicate a dwell and fixation of the users gaze on different parts of the medical data.

In some embodiments, the detected gazing pattern may be provided in the form of a heat map, where each region of the heatmap identifies a different region of the graphical representation of the medical data. Thus, a value associated with each region describes a length of time that the user has observed the respective region of the graphical representation of the medical data.

Of course, the detected gazing pattern may be provided/represented in a variety of different ways. The detected gazing pattern may equally be provided in a list form, with parts of the medical data associated with different observation time lengths and/or orders. Equally, the detected gazing pattern may simply describe whether or not the user has observed certain parts of the medical data.

To be clear, the gazing pattern does not need to be a visual map, nor relate to the order of observation. For example, the gazing pattern may be described by a set of rules, and whether the user has satisfied those rules. That is, the gazing pattern may simply be a list of actions that have been fulfilled by the user by observing the medical data. For instance, the list may be that the user has observed heart rate, blood pressure, and respiration rate values of the medical data for at least 1 second. Equally, the list may be that the user's gaze has been directed at the locations where heart rate, blood pressure, and respiration rate values were displayed for at least 1 second.

In some cases, the detected gazing pattern may describe a collection of gazing patterns, each relating to different observation events within an extended time period. That is, the detected gazing pattern may describe a time-varying eye gaze direction of the user observing a graphical representation of the medical data at a plurality of discrete time periods. Thus, whilst in some cases the detected gazing pattern may describe a time-varying eye gaze direction of the user during only a short time window (e.g., up to a minute), it may also describe a time-varying eye gaze direction of the user during a longer time window (e.g., up to an hour), during which the user observes the medical data multiple times (e.g., for multiple sub-minute periods during the hour). Accordingly, the detected gazing pattern may describe the gazing behavior of the user during each gazing event of the user in a long observation window.

The duration of the time window during which the eye gaze direction of the user is detected and described by the detected gazing pattern may be pre-defined. Alternatively, this duration may be determined based on the medical data being displayed. For example, if some clinically relevant information being displayed should be observed within an hour, then the time window may be up to an hour long (thus checking whether the user has observed the medical data in an anticipated manner within an hour). Alternatively, if some clinically relevant information being displayed should be observed as soon as possible (e.g., within a few minutes), then the time window may be shorter to reflect this.

Alternatively, the time window of the detected gazing pattern may be determined adaptively based on the differences between consecutive gazing patterns of the same user within the extended time window. For example, if it is noticed that (despite clinically relevant change in the displayed medical data) the user continues to observe the medical data in the same manner, then the time window may be shortened or the currently detected gazing pattern may be taken as the gazing pattern.

The gazing pattern may be detected using known means and techniques. Indeed, the skilled person would readily appreciate and apply a wide range of eye gaze tracking technology that may be used to detect a gazing pattern of a user observing a display.

In step 120, a deviation of the detected gazing pattern from an expected gazing pattern is determined. Similar to the detected gazing pattern, the expected gazing pattern describes a time-varying eye gaze direction of the user observing the graphical representation. However, the expected gazing pattern describes an expected or target time-varying gaze direction of the user. That is, the expected gazing pattern is indicative of how a user may be expected to observe the medical data.

For example, the expected gazing pattern may relate to how the user may be expected to observe the medical data considering the content of the medical data (e.g., focusing on interesting values, and only briefly observing nominal values), characteristics of the user (e.g., different users may be expected to observe different parts of the medical data, or spent more time on particular parts), or a context of the user whilst observing the medical data (e.g., the user may be time-pressured, or distracted by the environment).

In any case, the expected gazing pattern provides for how the user is expected to interact with the medical data. If all users were to perfectly comply with the expected gazing pattern, it may be the case that each user is provided with all information required to draw consistent conclusions from the medical data.

However, users do not always observe medical data as expected. Certain users may be distracted, or may not be completely familiar with, or adequately equipped for, the particular clinical situation described by the medical data. In other cases, users may suffer from tunnel-vision for various reasons. Accordingly, there is usually some variance in the way that users observe the same medical data. This can lead to different users drawing different conclusions from the same medical data, particularly if some users miss, or fail to spend sufficient time observing, important parts of the medical data.

Thus, step 120 provides that a deviation between the detected gazing pattern and the expected gazing pattern is determined/identified. This may be performed by simply comparing the two gazing patterns. For example, when both gazing patterns are represented in the form of heatmaps, one heatmap may be subtracted from the other to highlight parts of the medical data that have not been given adequate attention. Alternatively, key aspects of the medical data to be observed may be determined form the expected gazing pattern, and the detected gazing pattern analyzed in light of these key aspects.

In some cases, the deviation may describe a difference in actual and expected time spent by the user observing different parts of the medical data, and/or a difference in the actual and expected order in which the user observed the medical data, and/or a difference in the dwell and fixation for various parts of the medical data.

Put another way, depending on the quantification of the gazing patterns, different comparison techniques can be employed. For example, if the detected and expected gazing patterns are provided in the form of an image (like a heat map, with different color depending on the duration of the gaze), a distance between the first and second image can be computed. If the detected and expected gazing patterns are represented as a two-dimensional signal, where the first dimension shows the order and second dimension shows the time per regions of interest (ROI), the distance between the detected and expected signal can be computed. Furthermore, rules based criteria may be employed to determine the deviation.

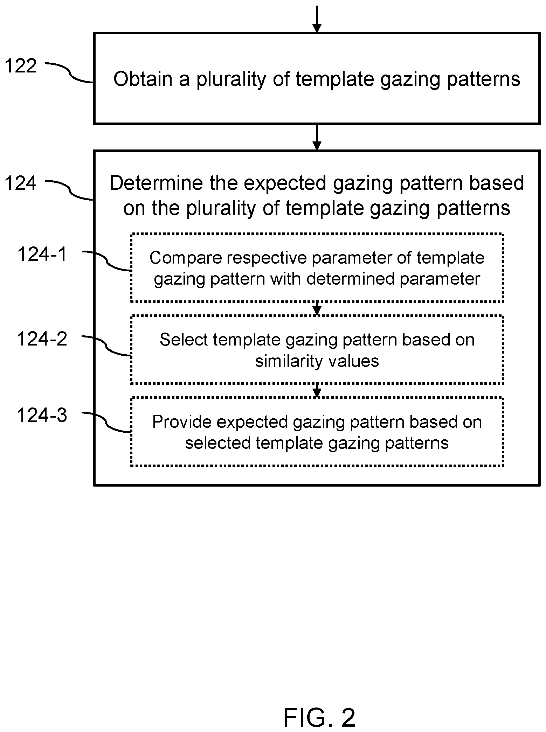

The expected gazing pattern may be obtained from a database/repository of expected gazing patterns, with the one most closely applying to the given graphical representation of medical data selected. Alternatively, the expected gazing pattern may be generated with a gazing pattern prediction model. The expected gazing pattern may be based on at least one of a status and/or condition of the subject(s) associated with the medical data, one or more characteristics of the user, and/or a contextual information associated with the user. More details on the expected gazing pattern are provided below in reference to Fig. 2.

In the case, as described above, that the detected gazing pattern describes a time-varying eye gaze direction of the user observing a graphical representation of the medical data at a plurality of discrete time periods (i.e., gazing behavior of the user during each gazing event of the user in a long observation window), the deviation from an expected gazing pattern may be determined with respect to the gaze of the user throughout this time window. That is, the deviation may be assessed with respect to the eye gaze pattern of the user during each discrete observation event as described by the detected gazing pattern.

For example, if the detected gazing pattern describes the eye gaze direction of the user during a one hour period, then the expected gazing pattern may be compared to the gazing pattern of the user during each observation event in the one hour time period described by the detected gazing pattern. If, at the end of this one hour time period, the user has not observed the medical data in the anticipated manner (according to the expected gazing pattern) during any observation event (or even a cumulation of observation events), then the graphical representation of the medical data presented to the user may be modified for the current or next gazing event.

In addition, if it is detected (according to the detected gazing pattern) that the user continues to observe the medical data in the same way during the time period of the detected gazing pattern, despite a change in the medical data being displayed, then this may indicate a deviation. That is, the user may be expected to change their gazing behavior when some clinically relevant medical data is displayed (e.g., a change in blood pressure or heart rate). If the detected gazing pattern describing eye gaze direction of the user over a number of discrete time windows indicates that no change in behavior has occurred, then this indicates that there is a deviation from an expected gazing pattern. Accordingly, the method may proceed to step 140 in which the graphical representation of the medical data is modified.

In (optional) step 130, the deviation is analyzed to determine a clinical relevance of the deviation. As explained above, the deviation describes a difference between how the user has observed the medical data, and how the user may be expected to observe the medical data. This difference may lead to a difference in conclusions drawn by different users, perhaps because a user missed a particularly important part of the medical data. However, in some cases, this difference may be immaterial. That is, the difference may not have a noticeable impact on the conclusions drawn by the user. Clinical relevance relates to the impact that the deviation may have on conclusions drawn by a medical professional.

By way of example, the expected gazing pattern may describe that the user is expected to focus on many lower-priority parts of the medical data only briefly, and on one high-priority part of the medical data for a long amount of time (e.g., because only one part of the medical data relates to a condition of the subject, with the rest of the information being superfluous). If the user were to spend longer looking at the high-priority part of the medical data than deemed necessary by the expected gazing pattern, then this may not be clinically relevant. Equally, if the user were to spend less time, or even no time at all, looking at lower-priority parts of the medical data than prescribed, this may also not be clinically relevant. In contrast, if the user were to linger on the lower-priority parts for a significant amount of time, and the high-priority part for an insufficient amount of time, then this may be considered clinically relevant.

It should be appreciated that the above is by way of example only. The clinical relevance of certain parts of the medical data may relate to the particular clinical situation, which may only be determine by analysis of the deviation. In some situations, only a small deviation may be required for clinical relevance, whereas in other situations large deviations may be required for clinical relevance.

If it is determined that the deviation is clinically relevant, then the method proceeds to step 140. If it is determined that the deviation is not clinically relevant, then the method may not proceed to step 140. Of course, this may not be completely binary, and a degree of clinical relevance may be used to alter the result of step 140 (i.e., changing a magnitude or mode by which the graphical representation is modified).

Put another way, if the determined clinical relevance satisfies a predetermined condition, then the graphical representation of medical data may be modified. The predetermined condition may describe the extent of clinical relevance required to justify modification of the graphical representation. The predetermined condition may be a preset condition of the method, or may be set by a healthcare provider or institution.

Of course, step 130 is optional. It may not be necessary to assess the clinical relevance, with any deviation in the detected gazing pattern being used as basis for modifying the representation of medical data. This may be simpler to implement, but may equally lead to unnecessary modifications of the graphical representation.

In step 140, the graphical representation of the medical data is modified. The modification is performed based on the deviation of the detected gazing pattern.

Modification of the graphical representation may include emphasizing certain parts of the medical data. For example, emphasizing parts of the graphical representation of the medical data may include highlighting, coloring, enlarging, italicizing, or boldening the graphical representation. Further, the graphical representation may be reorganized, or parts of the user interface may be altered so as to prompt the user to notice certain parts of the medical data. That is, there may a pointer or light provided to draw the users attention to certain parts of the medical data. Furthermore, an amount and type of medical data displayed may also be modified.

Equally, modification of the graphical representation may include de-emphasizing certain parts of the medical data. For example, de-emphasizing parts of the graphical representation of the medical data may include dulling, obscuring, shrinking, or blurring the graphical representation.

In certain embodiments, the graphical representation may also be altered so as to explicitly tell the user what they may have missed or focused too much on. That is, the graphical representation may explicitly inform the user as to the determined deviation of the detected gazing pattern.

In essence, the graphical representation of the medical data is altered so as to prompt the user to change their observation behavior in order to compensate for the determined deviation. Simply put, if the deviation indicates that the user has not adequately observed certain parts of the medical data, the modified graphical representation prompts the user to more closely observe these parts. Likewise, if the deviation indicates that the user has not observed the medical data in a prescribed/optimal order, the modified graphical representation prompts the user to observe parts of the medical data in order.

Although not presented, the method may further comprise steps of verifying the behavior of the user in order to ensure that they have observed the medical data in a way that is expected in response to the modification of the graphical representation.

Specifically, responsive to modifying the graphical representation, a prompted gazing pattern is detected. The prompted gazing pattern (like the detected gazing pattern) describes an eye gaze direction as a function of time of the user observing the graphical representation of the medical data. However, the prompted gazing pattern is detected in relation to the modified graphical representation of the medical data. Accordingly, the observation behavior of the user is expected to have changed. Nevertheless, the user may not have noticed the modification, or may not react in an anticipated manner.

Accordingly, a deviation of the prompted gazing pattern from a target gazing pattern describing a target eye gaze direction of the user responsive to the modification is determined. That is, a difference between the actual observation of the modified graphical representation and an anticipated/target/expected observation is determined. A clinical relevance of the deviation may be assessed to determine if action needs to be taken.

Then, the graphical representation of the medical data is modified again based on the determined deviation. Put simply, the provided graphical representation of the medical data may be modified repeatedly until it is detected that the user has observed the medical data in the desired manner. This may help to ensure that the user sees all important parts of the medical data to form an informed conclusion related to the medical data.

Fig. 2 is a flow diagram of a method for determining an expected gazing pattern according to an aspect of the invention. Focus will now be provided as to how to determine an expected gazing pattern. Nevertheless, known methods of determining an expected gazing pattern would be readily applied by the skilled person.

As described above, the expected gazing pattern describes an expected time-varying eye gaze direction of the user observing the graphical representation. Accordingly, the expected gazing pattern may be any time-varying eye gaze direction that is desirable considering the medical data of the graphical representation. For the purposes of the invention, the expected gazing pattern may be provided by any known means. That is, the expected gazing pattern may be selected from a database of expected gazing patterns, derived based on the medical data, and/or generated based on gazing data collected from one or more users.

For example, the expected gazing pattern may be based on a combination of one of a status and/or condition of the subject(s) associated with the medical data, one or more characteristics of the user, and/or a contextual information associated with the user. The status and/or condition of the subject(s) relate to the clinical state of the subject(s) described by the medical data (e.g., a heart condition, an unknown condition). The one or more characteristics of the user may comprise a role (e.g., nurse, surgeon), an experience level (e.g., novice, expert), a user identifier (e.g., an ID number indicating the specific user), and a user-subject familiarity (e.g., assigned nurse, new subject). The contextual information may comprise environmental information describing a surrounding environment of the user (e.g., a number of other users, a surrounding noise or movement), or time-related information describing time-based parameters associated with the user (e.g., a next appointment time).

Accordingly, in some embodiments the method may comprise a step of determining the expected gazing pattern based on a status and/or condition of a subject associated with the medical data. Additionally or alternatively, the method may comprise determining the expected gazing pattern based on a contextual information associated with the user. Additionally or alternatively, the method may comprise determining the expected gazing pattern based on one or more characteristics of the user.

It should be noted that the medical data may be analyzed to determine the status and/or condition of a subject associated with the medical data. Indeed, information about the condition and/or status of the subject will be embedded in the medical data, and therefor may be derived without external input. Similarly, the detected gazing pattern may be analyzed to determine the one or more characteristics of the user. That is, each user may have certain identifiers in their gazing pattern indicating who they are, their role, their level of expertise and their alertness levels.

The determination of the expected gazing pattern based on the above may be achieved by generating the expected gazing pattern using a gazing pattern prediction model. That is, the above parameters may be provided to the prediction model, and the prediction model may output the expected gazing pattern. Alternatively, or additionally, the gazing pattern prediction model may be provided with the graphical representation of the medical data, and may output the expected gazing pattern.

To summarize, when it is determined which user is currently observing the medical data, the medical data being presented may be analyzed to generate an expected gazing pattern. By way of example, if there is a change in blood pressure as described by the medical data, it may be determined that this is important and should be observed by the user, as this could indicate the advent of shock. Accordingly, the expected gazing pattern should reflect this. Another example is when the subject of the medical data is undergoing a certain workflow/protocol that suggest monitoring of a certain parameter, then the expected gazing pattern will include the observation of the certain parameter. The expected gazing pattern may depend upon: (i) prior eye gaze behavior; (ii) a role of the user; (iii) a familiarity level with subject; (iv) a relevant medical parameter derived from the subject condition or status; (v) sensor data of the subject; (vi) timing of observation of the medical data (e.g., is the current interaction taking longer than expected); (vii) a time of observation (e.g., a time of day, a time within a shift of the user, and/or a day of the week, etc.); and (viii) environment (e.g., is it busy, crowded, lots of distracting factors). Of course, further factors would be readily apparent to the skilled person.

In a further embodiment, the expected gazing pattern may be generated based on historical gazing data collected from one or more users. For example, gaze-related data and patient outcome data can be collected, and used to predict the gazing patterns that will result in a positive patient outcome. In some instances, a machine learning algorithm may be trained on gaze-related data and patient outcome data in order to be adapted to generate an expected gazing pattern based on real-time medical data.

In some examples, the machine learning algorithm may only be trained based on local data of the particular hospital and unit, as different organizations may have different protocols and modes of working. Nevertheless, data from similar units in other hospitals may also be used to train the machine learning algorithm, as long as the model is tuned/adapted based on the needs of the particular hospital/unit.

According to another embodiment, steps 122 and 124 of Fig. 2 may be provided.

In step 122, a plurality of template gazing patterns are obtained. Each template gazing pattern describes an expected time-varying eye gaze direction of a user observing medical data. Template gazing patterns may be pre-generated.

In step 124, the expected gazing pattern is determined based on the plurality of template gazing patterns. That is, the expected gazing pattern may be selected from the plurality of template gazing patterns, may be a mix of a subset of the template gazing patterns, or may be an edited version of one of the plurality of template gazing patterns. The determination may be made, for example, based on a similarity between the graphical representation of medical data associated with each template gazing pattern and the presently displayed graphical representation of medical data.

More particularly, in step 124-1, for each of the plurality of template gazing patterns, a respective status and/or condition associated with the template gazing pattern may be compared with the status and/or condition of the subject associated with the medical data to determine a similarity value between the template gazing pattern and the current situation. Additionally or alternatively, the respective characteristics of the user associated with the template gazing pattern may be compared with the characteristic of the user to determine a similarity value. Additionally or alternatively, the respective contextual information of the user associated with the template gazing pattern may be compared with the contextual information of the user to determine a similarity value

Accordingly, a similarity value is generated which indicates the situation of each template gazing pattern with the present situation. Then, in step 124-2, at least one of the plurality of template gazing patterns is selected based on similarity values of each of the template gazing patterns. Thus, the template gazing pattern(s) having the most similar situation may be selected.

Finally, in step 124-3, the expected gazing pattern is provided/determined based on the selected template gazing patterns. The expected gazing pattern may thus be chosen from one of the selected gazing patterns, may be a mix of the selected gazing patterns, or may be a modified version of one or more of the selected gazing patterns.

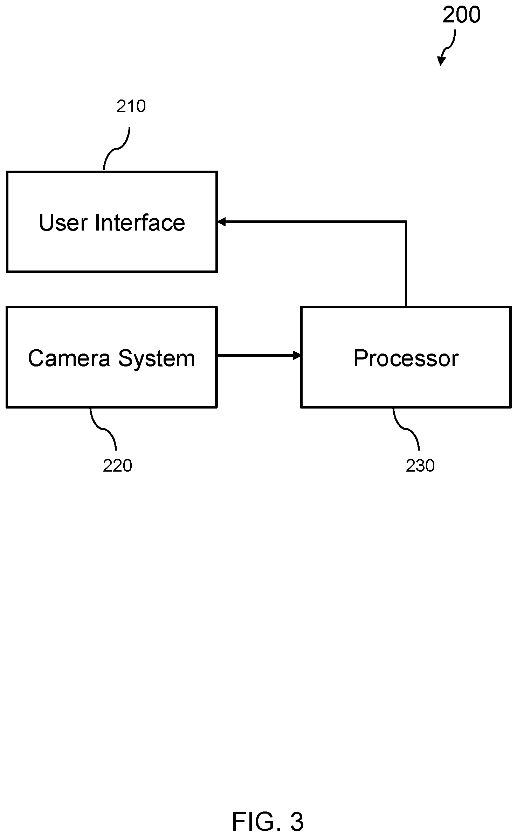

Moving on to Fig. 3, there is presented a simplified block diagram of a patient monitor according to another embodiment of the invention. The patient monitor may be suitable for conveying medical data of a single subject. Alternatively, the patient monitor may be suitable for conveying medical data of many subjects, for examples all subjects on a ward. The patient monitor may receive medical data from a plurality of sensors and other sources, process the medical data, and display/present the medical data for a user to observe.

According to the invention, the patient monitor 200 comprises a user interface 210, a camera system 220, and a processor 230. Each of these components may be provided as a single integrated device, or may be provided as separate but communicatively coupled devices.

The user interface 210 is configured to display a graphical representation of medical data. To this end, the user interface 210 may comprise one or more screens/displays for displaying the medical data. The user interface 210 may graphically represent the medical data in the form of graphs, charts, trend indicators, or raw numerals. The camera system/arrangement 220 is configured to detect a gazing pattern describing an eye gaze direction as a function of time of a user observing a graphical representation of the medical data. To this end, the camera system 220 may comprise one or more cameras for capturing an image of the user observing the medical data, and may process the image of the user to determine the gazing pattern of the user.

In some embodiments, the user interface 210 may be in the form of, or integrated in, an augmented reality device, virtual reality device, or mixed reality device. That is, the user interface 210 may be a screen or other display in an augmented or virtual reality device. Accordingly, the graphical representation of medical data may be displayed as part of the interface of the augmented or virtual reality device.

In this case, the camera system/arrangement 220 may be integrated in the augmented, virtual or mixed reality device. For example, the augmented, virtual or mixed reality device may comprise a headset that has one or more cameras that track the gaze of a user observing an interface displayed by the device. Accordingly, the invention may apply to a user using an augmented, virtual or mixed reality device to observe medical data of a subject.

The processor 230 is configured to determine a deviation of the detected gazing pattern from an expected gazing pattern. Accordingly, the processor 230 receives the detected gazing pattern from the camera system 220, and processes the detected gazing pattern and the expected gazing pattern to determine the deviation. The expected gazing pattern may be pre-stored in the processor 230 or other storage. Alternatively, as described in reference to Fig. 2, the expected gazing pattern may be generated/determined by the processor 230.

Furthermore, the processor 230 is configured to modify the graphical representation of medical data based on the deviation. That is, the processor 230 may control the user interface 210 in such a way so as to modify the graphical representation of the medical data displayed on the user interface 210.

Of course, the patient monitor 200 may employ any method described in relation to Figs 1 and 2. As a result, the patient monitor 200 may prompt the user observing the medical data in such a way that they are more likely to conform to an expected gazing pattern, and may thus view all important aspects of the medical data. In turn, this means that there may be improved consistency and accuracy of conclusions drawn based on the displayed medical data by different users.

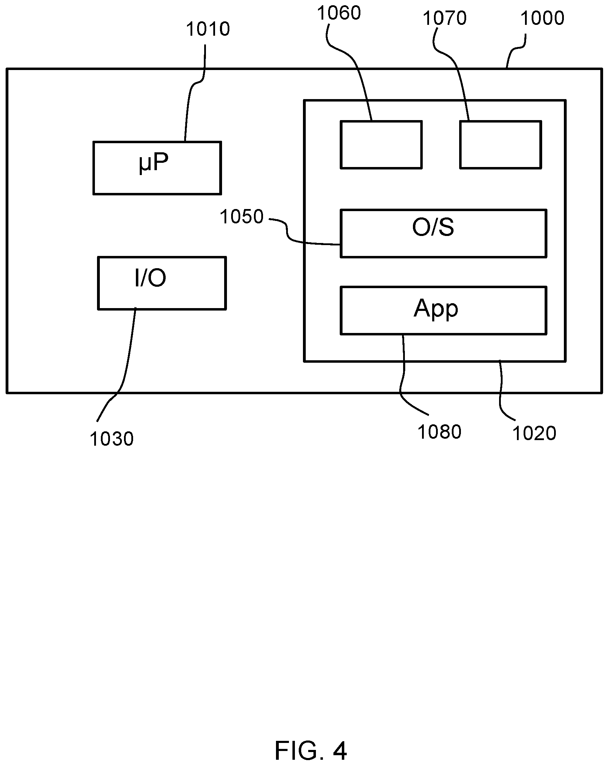

Fig. 4 illustrates an example of a computer 1000 within which one or more parts of an embodiment may be employed. Various operations discussed above may utilize the capabilities of the computer 1000. For example, one or more parts of a system for acquiring an input from a user to control an interface may be incorporated in any element, module, application, and/or component discussed herein. In this regard, it is to be understood that system functional blocks can run on a single computer or may be distributed over several computers and locations (e.g. connected via internet).

The computer 1000 includes, but is not limited to, smart phones, PCs, workstations, laptops, PDAs, palm devices, servers, storages, and the like. Generally, in terms of hardware architecture, the computer 1000 may include one or more processors 1010, memory 1020 and one or more I/O devices 1030 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 1010 is a hardware device for executing software that can be stored in the memory 1020. The processor 1010 can be virtually any custom made or commercially available processor, a central processing unit (CPU), a digital signal processor (DSP), or an auxiliary processor among several processors associated with the computer 1000, and the processor 1010 may be a semiconductor-based microprocessor (in the form of a microchip) or a microprocessor.

The memory 1020 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 1020 may incorporate electronic, magnetic, optical, and/or other types of storage media. Note that the memory 1020 can have a distributed architecture, where various components are situated remote from one another, but can be accessed by the processor 1010.

The software in the memory 1020 may include one or more separate programs, each of which comprises an ordered listing of executable instructions for implementing logical functions. The software in the memory 1020 includes a suitable operating system (O/S) 1050, compiler 1060, source code 1070, and one or more applications 1080 in accordance with exemplary embodiments. As illustrated, the application 1080 comprises numerous functional components for implementing the features and operations of the exemplary embodiments. The application 1080 of the computer 1000 may represent various applications, computational units, logic, functional units, processes, operations, virtual entities, and/or modules in accordance with exemplary embodiments, but the application 1080 is not meant to be a limitation.

The operating system 1050 controls the execution of other computer programs, and provides scheduling, input-output control, file and data management, memory management, and communication control and related services. It is contemplated by the inventors that the application 1080 for implementing exemplary embodiments may be applicable on all commercially available operating systems.

Application 1080 may be a source program, executable program (object code), script, or any other entity comprising a set of instructions to be performed. When a source program, then the program is usually translated via a compiler (such as the compiler 1060), assembler, interpreter, or the like, which may or may not be included within the memory 1020, so as to operate properly in connection with the O/S 1050. Furthermore, the application 1080 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, functional programming and the like.

The I/O devices 1030 may include input devices such as, for example but not limited to, a mouse, keyboard, scanner, microphone, camera, etc. Furthermore, the I/O devices 1030 may also include output devices, for example but not limited to a printer, display, etc. Finally, the I/O devices 1030 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 1030 also include components for communicating over various networks, such as the Internet or intranet.

If the computer 1000 is a PC, workstation, intelligent device or the like, the software in the memory 1020 may further include a basic input output system (BIOS) (omitted for simplicity). The BIOS is a set of essential software routines that initialize and test hardware at start-up, start the O/S 1050, and support the transfer of data among the hardware devices. The BIOS is stored in some type of read-only-memory, such as ROM, PROM, EPROM, EEPROM or the like, so that the BIOS can be executed when the computer 1000 is activated.

When the computer 1000 is in operation, the processor 1010 is configured to execute software stored within the memory 1020, to communicate data to and from the memory 1020, and to generally control operations of the computer 1000 pursuant to the software. The application 1080 and the O/S 1050 are read, in whole or in part, by the processor 1010, perhaps buffered within the processor 1010, and then executed.

When the application 1080 is implemented in software it should be noted that the application 1080 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 1080 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The methods described in relation to Figs 1 and 2, and the system described in relation to Fig. 3, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagram of Fig. 3 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

## Claims

1. A method for displaying medical data, comprising:
detecting (110) a gazing pattern describing a time-varying eye gaze direction of a user observing a graphical representation of the medical data;
determining (120) a deviation of the detected gazing pattern from an expected gazing pattern describing an expected time-varying eye gaze direction of the user observing the graphical representation; and
modifying (140), based on the deviation of the detected gazing pattern, the graphical representation of the medical data.

2. The method of claim 1, further comprising:
obtaining a plurality of template gazing patterns, each template gazing pattern describing an expected time-varying eye gaze direction of a user observing medical data; and
determining the expected gazing pattern based on the plurality of template gazing patterns.

3. The method of claim 1 or 2, further comprising determining the expected gazing pattern based on a status and/or condition of a subject associated with the medical data.

4. The method of claim 3, wherein determining the expected gazing pattern comprises:
obtaining a plurality of template gazing patterns, each template gazing pattern describing an expected time-varying eye gaze direction of a user observing medical data of a subject with a respective status and/or condition;
for each of the plurality of template gazing patterns, comparing the respective status and/or condition associated with the template gazing pattern with the status and/or condition of the subject associated with the medical data to determine a similarity value; and
selecting at least one of the plurality of template gazing patterns based on similarity values of each of the template gazing patterns; and
providing the expected gazing pattern based on the selected template gazing patterns.

5. The method of claim 3, wherein determining the expected gazing pattern comprises generating, with a gazing pattern prediction model, the expected gazing pattern based on the status and/or condition of the subject associated with the medical data.

6. The method of any of claims 3-5, further comprising analyzing the medical data to determine the status and/or condition of a subject associated with the medical data.

7. The method of any of claims 1-6, further comprising determining the expected gazing pattern based on one or more characteristics of the user, and optionally wherein the one or more characteristics comprise a role, an experience level, a user identifier, and a user-subject familiarity.

8. The method of claim 7, wherein determining the expected gazing pattern comprises:
obtaining a plurality of template gazing patterns, each template gazing pattern describing an expected time-varying eye gaze direction of a different user observing medical data, each different user having one or more characteristics;
for each of the plurality of template gazing patterns, comparing the respective characteristics of the user associated with the template gazing pattern with the characteristic of the user to determine a similarity value; and
selecting at least one of the plurality of template gazing patterns based on similarity values of each of the template gazing patterns; and
providing the expected gazing pattern based on the selected template gazing patterns.

9. The method of claim 7 or 8, further comprising analyzing the detected gazing pattern to determine the one or more characteristics of the user.

10. The method of any of claims 1-9, further comprising determining the expected gazing pattern based on a contextual information associated with the user, and optionally wherein the contextual information comprises environmental information describing a surrounding environment of the user, or time-related information describing time-based parameters associated with the user.

11. The method of any of claims 1-10, further comprising analyzing (130) the deviation to determine a clinical relevance of the deviation, and wherein the graphical representation of the medical data is modified (140) responsive to the clinical relevance satisfying a predetermined condition.

12. The method of any of claims 1-11, further comprising:
detecting, responsive to modifying the graphical representation, a prompted gazing pattern describing an eye gaze direction as a function of time of the user observing the modified graphical representation; and
determining a deviation of the prompted gazing pattern from a target gazing pattern describing a target eye gaze direction of the user responsive to the modification; and
modifying, based on the deviation of the prompted gazing pattern, the graphical representation of the medical data.

13. The method of any of claims 1-12, wherein the detected gazing pattern and the expected gazing pattern are each represented as heat maps, wherein each region of the heatmap identifies a different region of the graphical representation, and wherein a value associated with each region describes a length of time that the user has observed the respective region of the graphical representation.

14. A computer program comprising computer program code means adapted, when said computer program is run on a computer, to implement the method of any of claims 1-13.

15. A patient monitor (200), comprising:
a user interface (210) configured to display a graphical representation of medical data;
a camera system (220) configured to detect a gazing pattern describing an eye gaze direction as a function of time of a user observing a graphical representation of the medical data; and
a processor (230) configured to:
determine a deviation of the detected gazing pattern from an expected gazing pattern, and
modify, based on the deviation of the detected gazing pattern, the graphical representation of the medical data.
